(19) Europäisches Patentamt
European Patent Office
Office européen des brevets

(11) **EP 0 669 133 B1**

(12) **EUROPÄISCHE PATENTSCHRIFT**

(45) Veröffentlichungstag und Bekanntmachung des Hinweises auf die Patenterteilung:
21.05.1997 Patentblatt 1997/21

(51) Int. Cl.⁶: $A61K\ 35/76$, $A61K\ 39/39$, $C12P\ 21/00$, $C12N\ 7/00$

(21) Anmeldenummer: 94103047.0

(22) Anmeldetag: 01.03.1994

(54) **Paramunitätsinducer auf der Basis von Kombinationen von Pockenviruskomponenten, Verfahren zu ihrer Herstellung und ihre Verwendung als Arzneimittel**

Paramunity inducer based ou combinations of poxvirus components, process to prepore it and its use as medicament

Inducteur paramunitaire à base de combinaisons de composants du poxvirus, procédé de préparation et son utilisation comme médicament

(84) Benannte Vertragsstaaten:
AT BE CH DE DK ES FR GB GR IE IT LI LU MC NL PT SE
Benannte Erstreckungsstaaten:
SI

(30) Priorität: 23.02.1994 DE 4405841

(43) Veröffentlichungstag der Anmeldung:
30.08.1995 Patentblatt 1995/35

(83) Erklärung nach Regel 28(4) EPÜ
(Sachverständigenlösung)

(73) Patentinhaber: **Mayr, Anton, Prof. Dr.med.vet. Dr.h.c.mult.**
**D-80992 München (DE)**

(72) Erfinder: **Mayr, Anton, Prof. Dr.med.vet. Dr.h.c.mult.**
**D-80992 München (DE)**

(74) Vertreter: **Vossius, Volker, Dr. et al**
**Dr. Volker Vossius,**
**Patentanwaltskanzlei - Rechtsanwaltskanzlei,**
**Holbeinstrasse 5**
**81679 München (DE)**

(56) Entgegenhaltungen:
EP-A- 0 312 839

- **ZENTRALBLATT FÜR VETERINÄRMEDIZIN, REIHE B Bd. 28, Nr. 7 , 1981 , BERLIN Seiten 535 - 552 MAYR, A. ET AL 'Bekämpfung des Ecthyma contagiosum (Pustulardermatitis) der Schafe mit einem neuen Parenteral-Zellkultur-Lebendimpfstoff'**
- **ZENTRALBLATT FÜR VETERINÄRMEDIZIN Bd. 33, Nr. 5 , 1986 , BERLIN Seiten 321 - 339 MAYR, A. ET AL 'Vergleichende Untersuchungen über die immunostimulierende (paramunisierende) Wirksamkeit von BCG, Levamisol, Corynebacterium parvum und Präparaten aus pockenviren in verschiedenen "in vivo"-Testen'**
- BIOLOGICAL ABSTRACTS, Band 80, 1985, Seite AB-438, Zusammenfassung Nr.13095, Mayr, A. **Paramunity and paramunization**
- **Medical Dictionary of the English and German Languages, p.569**
- **Pschyrembel-Klinisches Wörterbuch, p.1253**
- **Roche Lexikon Medizin, pages 3 and 1309**
- **Kurzes Lehrbuch der Immunologie, page 361**
- **Lexikon der Immunologie, pages 642-645**
- **Handbuch der Schutzimpfungen in der Tiermedizin, pages 82-85**
- **The Medical and Pharmaceutical Dictionary, page 1**
- **Hexal-Wörterbuch Medizin, page 1**
- **Wörterbuch Klinische Medizin, page 2**

## Beschreibung

Die vorliegende Erfindung betrifft multipotente Paramunitätsinducer auf der Basis von Kombinationen von Pockenviruskomponenten, Verfahren zu ihrer Herstellung und ihre Verwendung als Arzneimittel.

Das Immunsystem der Warmblüter, insbesondere das der Säuger und Vögel, besteht aus einem antigenspezifischen sowie einem antigenunspezifischen Teil. Beide Teile sind miteinander vernetzt und bilden dadurch ein einheitliches Organsystem. Die antigenspezifischen Mechanismen sind für den Aufbau einer Immunität, die antigenunspezifischen für den Aufbau der Paramunität verantwortlich. Entsprechend wird der antigenunspezifische Teil der Abwehr aus historischen wie funktionellen Gründen als paraspezifisches Immunsystem bezeichnet. Die immunologische Forschung hat sich bisher hauptsächlich mit dem antigenspezifischen Teil des Immunsystems, d.h. mit der Immunitätsbildung, befaßt. Die Nutzung dieses Abwehrpotentials führte z.B. zur Entwicklung der aktiven und passiven Schutzimpfung. Die Nutzung der paraspezifischen Aktivitäten des Immunsystems für die Prophylaxe und Therapie steht dagegen noch in den Anfängen. Die paraspezifische Abwehr ermöglicht es dem Organismus, sich bei Konfrontation mit den unterschiedlichsten Fremdstoffen, Infektionserregern, Toxinen und transformierten körpereigenen Zellen sofort zu wehren. Zwischen den paraspezifischen und den spezifischen Aktivitäten des Immunsystems bestehen enge funktionelle Wechselwirkungen, wobei der Informationsfluß in der Regel von den zuerst reagierenden paraspezifischen zu den später einsetzenden spezifischen Mechanismen des Immunsystems läuft. Die paraspezifische Abwehr des Organismus kann auf diese Weise bei Infektionen mit besonders virulenten Pathogenen die Zeit bis zur Ausbildung einer spezifischen Immunität überbrücken.

Seit Einführung der Schutzimpfung gegen die Variola durch E. Jenner im Jahre 1798 mit einem vom Tier (Rind, Pferd) gewonnenen Impfstoff auf der Basis des Vacciniavirus wird über empirisch gewonnene Ergebnisse berichtet, die zeigten, daß durch die Pokken-Schutzimpfung andere Infektionen und Krankheiten, bevorzugt chronische und rezidivierende, an denen die Impflinge zum Zeitpunkt der Impfung zufällig litten, überraschend schnell bzw. ohne Komplikationen abheilten. Insbesondere betrifft dies Herpesinfektionen unterschiedlicher Genese, Papillome, chronische Ekzeme und Erkrankungen des Hals-, Nasen- und Ohrenbereiches. Andererseits beobachtete man bei den Impflingen eine kurzzeitige, generell erhöhte Widerstandsfähigkeit gegen akute Infektionen der Umgebung. Ähnliche Phänomene sind nach Schutzimpfungen gegen Tierpocken bekannt. Aus diesen Befunden folgerte man, daß Pockenviren bzw. bestimmte Strukturkomponenten dieser Viren unspezifisch die Widerstandsfähigkeit des Organismus gegenüber Infektionen und Tumoren günstig beeinflussen können. Da diese unspezifischen Heilungsvorgänge sofort nach der Schutzimpfung und damit 5-7 Tage vor, aber auch noch parallel zur Bildung der spezifischen Impfimmunität einsetzen, bezeichnete A. Mayr 1978 diese unspezifischen Auswirkungen einer Schutzimpfung als "paraspezifisch". Medikamente, die speziell zur Nutzung derartiger paraspezifischer Wirkungen hergestellt werden, bezeichnet man dementsprechend als "Paramunitätsinducer"; vgl. A. Mayr, Prämunität, Prämunisierung und paraspezifische Wirkung von Schutzimpfungen, Münch. Med. Wschr. 1978, Band 120, S. 239 bis 242.; Mayr, A., H. Raettig, H. Stickl und M. Alexander, 1979: Paramunität, Paramunisierung, Paramunitätsinducer. Fortschr. Med. 97, 1159-1165 und 1205-1210.

Aus der DE-OS 27 14 665 bzw. der US-PS 4 191 745 sind Präparate zur Behandlung von Herpes zoster und anderen Herpesinfektionen auf der Basis von Pokkenviren bekannt. Für die Veterinärmedizin wurden entsprechend Paramunitätsinducer aus gereinigten, attenuierten und inaktivierten Avipockenviren und Parapockenviren entwickelt. Diese Paramunitätsinducer sind als "Duphapind [R]" bzw. "Duphamun [R]" (PIND-AVI) bzw. "Baypamun [R]" (PIND-ORF) für praktisch alle Nutz- und Heimtierarten in den europäischen Ländern zugelassen. Der Paramunitätsinducer PIND-AVI wird aus einem attenuierten Hühnerpockenvirus, Stamm HP-1, der Paramunitätsinducer PIND-ORF aus einem attenuierten Parapockenvirus, Stamm D-1701 hergestellt. Die Inaktivierung der attenuierten Viren erfolgt in an sich bekannter Weise, z.B. durch $\gamma$-Bestrahlung oder auf chemischem Wege, wie etwa durch Behandlung mit $\beta$-Propiolacton.

Die in der Humanmedizin zur Immunstimulierung und als "adjuvante Immuntherapie" bevorzugt verwendeten Medikamente mit paramunisierenden Eigenschaften sind BCG (Bacillus Calmette Guerin-Keime), Levamisol und *Corynebacterium parvum* (syn. *Propionibacterium acnes*). Diese Präparate sind jedoch den auf Pockenviren basierenden Paramunitätsinducern in bezug auf ihre paramunisierende Wirkung unterlegen, wie sich aus den in Tabelle 1 zusammengefaßten Ergebnissen ergibt, die in Vergleichsuntersuchungen dieser Präparate mit PIND-AVI und PIND-ORF im VSV-Babymaus-Test erhalten wurden; vgl. Mayr, A., M. Büttner, S. Pawlas, V. Erfle, B. Mayr, R. Brunner und K. Osterkorn, 1986: Vergleichende Untersuchungen über die immunstimulierende (paramunisierende) Wirksamkeit von BCG, Levamisol, *Corynebacterium parvum* und Präparaten aus Pockenviren in verschiedenen "*in vivo*"- und "*in vitro*"-Testen, J. Vet. Med. B 33, 321-339.

Der vorliegenden Erfindung liegt die Aufgabe zugrunde, multipotente Paramunitätsinducer auf der Basis von Pockenviruskomponenten für die Humanmedizin und Veterinärmedizin bereitzustellen, die bezüglich ihrer Potenz und bezüglich ihrer an der Paramunisierung beteiligten Aktivitäten verbessert sind. Eine weitere Aufgabe der vorliegenden Erfindung besteht ferner darin, ein verbessertes Verfahren zur Herstellung derartiger multipotenter Paramunitätsinducer durch eine neue Technik der Virusvermehrung in Zellkulturen und/oder -inaktivierung bereitzustellen. Eine weitere Aufgabe der Erfindung besteht schließlich darin, pharmazeutische Zusammensetzungen zur Verwendung als Arzneimittel auf der Basis dieser multipotenten Paramunitätsinducer bereitzustellen.

Entsprechend betrifft die Erfindung multipotente Paramunitätsinducer auf der Basis einer Kombination zweier oder mehrerer Pockenviruskomponenten, die sich von unterschiedlichen Pockenvirusstämmen mit paramunisierenden Eigenschaften ableiten. Die Erfindung betrifft weiter multipotente Paramunitätsinducer auf der Basis einer Kombination mehrerer unterschiedlicher Spezies von inaktivierten, attenuierten Pockenviren mit paramunisierenden Eigenschaften. Die Erfindung betrifft ferner ein Verfahren zur Herstellung von Paramunitätsinducern, bei dem zwei oder mehrere Pockenviruskomponenten kombiniert werden, die sich von unterschiedlichen Pockenvirusstämmen mit paramunisierenden Eigenschaften ableiten. Die Erfindung betrifft schließlich Verfahren zur Herstellung der erfindungsgemäßen multipotenten Paramunitätsinducer sowie pharmazeutische Zusammensetzungen zur Verwendung als Arzneimittel, die diese multipotenten Paramunitätsinducer umfassen.

Überraschend wurde dabei gefunden, daß die Kombination von Pockenviruskomponenten in den erfindungsgemäßen multipotenten Paramunitätsinducern nicht zur Verminderung oder gar zum Verlust der jeweiligen paramunisierenden Aktivitäten der einzelnen Pockenviruskomponenten führt. Vielmehr wurde festgestellt, daß durch die Kombination der von verschiedenen Pockenvirenstämmen abgeleiteten Pockenviruskomponenten in den erfindungsgemäßen multipotenten Paramunitätsinducern nicht nur eine Addierung oder Ergänzung, sondern eine Potenzierung der jeweiligen paramunisierenden Wirkung zustande kommt. Experimente haben gezeigt, daß die Wirkung der aus Pockenviruskomponenten kombinierten multipotenten Paramunitätsinducern weit über den jeweiligen Einzelwirkungen liegt. Dieser Effekt war nicht vorhersehbar und verbessert derartige Paramunitätsinducer gegenüber bekannten Präparaten aus einer einzigen Pockenviruskomponente bezüglich Ihrer Potenz und bezüglich ihrer an der Paramunisierung beteiligten Aktivitäten. Ferner haben die erfindungsgemäßen multipotenten Paramunitätsinducer überraschenderweise praktisch keine immunogenen, jedoch sehr starke paramunisierende Eigenschaften, so daß mehrmalige und kontinuierliche Applikationen möglich und unschädlich sind.

Die Erfindung basiert ferner auf dem überraschenden Befund, daß zwischen den Epitopen der Strukturproteine der Pockenviren, die für die Paramunisierung und jenen, die für die Immunisierung verantwortlich sind, eine Konkurrenzsituation besteht. Je stärker die Aktivität der für die antigenspezifische Immunisierung verantwortlichen Epitope abnimmt, um so mehr steigt die paraspezifische Aktivität an. Dies läßt sich durch zwei Beobachtungen belegen:

a) Durch die Attenuierung über mehrere 100 Passagen in Zellkulturen nehmen die immunisierenden Eigenschaften von Pockenviren ab, während die paraspezifischen Aktivitäten nicht nur ansteigen, sondern bei bestimmten Pockenstämmen erst nach der Attenuierung auftreten;

b) durch Inaktivierung der zur Herstellung der Paramunitätsinducer geeigneten Pockenviren, vorzugsweise durch Bestrahlung, Hitze- oder pH-Einwirkung oder besonders bevorzugt durch eine chemische Behandlung mit β-Propiolacton unter speziellen Bedingungen, verlieren die Pockenviren ihre immunisierenden Eigenschaften während die paramunisierenden Aktivitäten ansteigen.

Die erfindungsgemäßen Kombinationen der Pockenviruskomponenten sind zur Verwendung als multipotente Paramunitätsinducer sowohl in der Humanmedizin als auch in der Tiermedizin geeignet.

Der Ausdruck "Pockenviruskomponente", wie er im Zusammenhang mit dieser Erfindung verwendet wird, umfaßt eine Vielzahl viraler Strukturen, die von Pockenviren mit paramunisierenden Eigenschaften abgeleitet sind, beispielsweise vermehrungsfähige oder inaktivierte frisch isolierte Pockenviren, vermehrungsfähige oder inaktivierte rekombinierte Pockenviren, die sich von frisch isolierten Pockenviren ableiten, vermehrungsfähige oder inaktivierte attenuierte Pockenviren, vermehrungsfähige oder inaktivierte rekombinierte Pockenviren, die sich von attenuierten Pockenviren ableiten, die abgetrennten Hüllen sowie Spaltprodukte und aberrante Formen dieser Hüllen der vorstehend aufgezählten Pockenviren, einzelne virale Polypeptide, die durch biochemische oder immunochemische Methoden von Kulturen gewonnen wurden, die mit den vorstehend aufgezählten Pockenviren infiziert wurden sowie mittels prokaryontischer oder eukaryontischer Expression gewonnene rekombinante virale Polypeptide, die sich wenigstens teilweise von einem oder mehreren der viralen Polypeptide der vorstehend aufgezählten Pockenviren ableiten.

Der Ausdruck "Kombination", wie er im Zusammenhang mit dieser Erfindung verwendet wird, umfaßt sowohl das Mischen zweier oder mehrerer einzelner Pockenviruskomponenten, die sich von verschiedenen Pockenvirusstämmen ableiten, als auch die Kombinierung mehrerer solcher einzelner Pockenviruskomponenten in einem Polypeptid oder Polypeptidkomplex. Von Pockenviren abgeleitete Komponenten, wie rekombinierte Pockenviren, einzelne virale Polypeptide oder rekombinante virale Polypeptide, die infolge von Mutation oder genetischer Manipulation zwei oder mehrere Polypeptidsequenzen umfassen, die sich von zwei oder mehreren Pockenvirusstämmen ableiten, werden folglich ungeachtet der Tatsache, daß diese Polypeptidsequenzen Teile einer einzigen Aminosäurekette bilden, so betrachtet, als handelte es sich um zwei oder mehrere individuelle Pockenviruskomponenten. Dies gilt in gleicher Weise für einzelne Pockenviruskomponenten, die in irgendeiner Weise physikalisch oder chemisch miteinander verbunden sind.

Der Ausdruck "multipotent", wie er im Zusammenhang mit dieser Erfindung verwendet wird, meint die vielfältigen Anwendungsmöglichkeiten der erfindungsgemäßen Paramunitätsinducer in bezug auf Prophylaxe und Therapie, z.B. bei folgenden Indikationen:

1) Infektiöse Faktorenkrankheiten und Mischinfektionen, chronische Manifestationen infektiöser Prozesse, hartnäckig rezidivierende Infektionen und Chemotherapie-resistente, bakterielle und virale Infektionen,

2) Abwehrschwächen bzw. Dysregulationen im Abwehrsystem eines Organismus,

3) neonatale Infektionsbedrohung,

4) adjuvante Therapie bei bestimmten Tumorkrankheiten bzw. Verhütung der Metastasierung,

5) Regulierung der Homöostase zwischen Hormon-, Kreislauf-, Stoffwechsel- und Nervensystem.

Zur Prophylaxe sind die Paramunitätsinducer insbesondere geeignet in folgenden Fällen:

- Zur schnellen Aktivierung der Abwehr bei Neugeborenen
- Vor zu erwartenden Streß-Situationen (Standortwechsel, psychische Belastung, Hochleistung u.a.m.)
- Vor Hospitalisierung
- Bei akuter Infektionsgefährdung
- Zur Verhütung von Impfkomplikationen
- Zur Verminderung der Gefahr von Tumoren bzw. ihrer Metastasierung
- Zur Unterstützung der Bioregulation
- Zur Steigerung der Leistung
- Zur Erhöhung der Lebenserwartung
- Zur Regulierung der Homöostase im Verbund mit dem Hormon-, Kreislauf-, Stoffwechsel- und Nervensystem.

Für die Therapie sind die Paramunitätsinducer insbesondere geeignet in folgenden Fällen:

- Bei Immunschwächen
- Bei Viruskrankheiten, therapieresistenten bakteriellen Krankheiten und infektiösen Faktorenkrankheiten
- Bei chronischen und rezidivierenden Infektionen
- Bei Tumoren
- In der Rekonvaleszenz
- Bei Chemo- und Antibiotikatherapie
- Bei Leberkrankheiten unterschiedlicher Genese
- Bei chronischen Hautkrankheiten

- Bei immunpathogenen Folgekrankheiten.

Tabelle 1 zeigt die paramunisierenden Wirkungen verschiedener Pockenviruskomponenten sowie Paramunitätsinducer im VSV-Babymaus-Test. Die Zahlenwerte stehen für die Wirkungsindizes. Die Methodik ist in der DE-OS 27 14 665 beschrieben.

Tabelle 2 zeigt die Potenzierung der paramunisierenden Wirksamkeit verschiedener Pockenvirusstämme im VSV-Test in Abhängigkeit vom Grad der Attenuierung. Verwendet wurden inaktivierte Virussuspensionen.

Tabelle 3 zeigt den Einfluß der Attenuierung auf die Induktion der Interferonsynthese mononukleärer Leukozyten des peripheren Blutes verschiedener Spezies.

Tabelle 4 zeigt die Verstärkung der paramunisierenden Wirkung eines multipotenten Paramunitätsinducers auf der Basis eines rekombinierten Pockenvirus gegenüber der Wirkung der Einzelkomponenten.

Abbildung 1 zeigt einen Vergleich der Wirkungsindizes aus 2 VSV-Testen mit attenuiertem Hühnerpockenvirus HP1, Parapockenvirus ORF D1701 und einem Kombinationspräparat aus HP1 und D1701.

Abbildung 2 zeigt die potenzierende Wirkung einer erfindungsgemäßen Kombination der Pockenviruskomponenten ORF-Protein-4D9 und PIND-AVI gegenüber der Wirkung der beiden Einzelkomponenten auf die NK (natürliche Killerzellen)-Zellaktivität im Chromium 51-Freisetzungstest. Die Werte entsprechen dem Mittel aus zwei unterschiedlichen Versuchsansätzen. Die Testmethodik ist beschrieben bei Mayr 1986, a.a.O.

Zur Verwendung als Pockenviruskomponenten für die erfindungsgemäßen multipotenten Paramunitätsinducer können Pockenvirusspezies als frisch isolierte, sog. virulente Feldstämme oder in attenuierter, avirulenter Form verwendet werden. Aufgrund der im Laufe der Attenuierung gegenüber nicht attenuierten Stämmen steigenden paramunisierenden Eigenschaften und aufgrund von Sicherheitserwägungen ist die Verwendung von attenuierten Stämmen bevorzugt. Hierfür werden die betreffenden Virusstämme in Serienpassagen in Zellkulturen vermehrt. Durch regelmäßig dazwischen geschaltete Klonselektion mittels Plaquetechnik wird genetisch einheitliches Virusmaterial gewonnen, das sich durch Mutation und Selektion den Kulturbedingungen besonders gut angepaßt hat. Der Attenuierungsgrad wird im Verlauf der Passagen durch Verimpfung auf empfängliche Wirte (z.B. Federfollikelmethode bei Küken, Kanarienvogel etc., Skarifizierung der Kaninchenhaut etc.) überprüft. Die einzelnen Nachweisverfahren sind bekannt; vgl. Mayr, A. und E. Munz, 1964: Veränderungen von Vaccinevirus durch Dauerpassagen in Hühnerembryofibroblasten-Kulturen. Zbl. Bakt. Hyg. I. Orig. 195, 24-35; Mayr, A., F. Hartig und I. Bayr, 1964: Entwicklung eines Impfstoffes gegen die Kanarienpocken auf der Basis eines attenuierten Kanarienpocken-Kulturvirus. Zbl. Vet. Med. B 12, 41-49; Mayr, A. und K. Malicki, 1966: Attenuierung von virulentem Hühnerpockenvirus in Zellkulturen und Eigenschaften des attenuierten Virus. Zbl. Vet. Med. B 13, 1-13; Mayr, A., M. Herlyn, H. Mahnel, A. Danco, A. Zach und H. Bostedt, 1981: Bekämpfung des Ecthyma contagiosum (Pustulardermatitis) der Schafe mit einem neuen Parenteral-Zellkultur-Lebendimpfstoff. Zbl. Vet. Med. B 28, 535-552. Normalerweise werden mehrere unterschiedliche Methoden zur Bestimmung des Attenuierungsgrades der betreffenden Pockenvirusspezies benutzt.

Die Serienpassagen zur Attenuierung werden üblicherweise durch eine dreimalige Klonselektion mittels Plaque-Endverdünnungs-Technik abgeschlossen. Damit wird gewährleistet, daß das verwendete Virusmaterial genetisch einheitlich ist. Alle nachstehend genannten Pockenvirusstämme wurden nach diesem Prinzip gewonnen.

Für die Attenuierung von Hühnerpockenvirus werden 200 bis 500 Kulturpassagen, bevorzugt mindestens 440 Passagen benötigt. Für die Attenuierung von Parapockenvirus sind 100 bis 200 Kulturpassagen, bevorzugt mindestens 170 gestufte Passagen, zuerst in embryonalen Lammnieren-Kulturen gefolgt von Passagen in embryonalen Kälberlungen-Kulturen und in Zellinien von Affennieren (z.B. MA, Vero) erforderlich. Das MVA-Vaccinia-Virus benötigt eine Attenuierung über 400 bis 600 Kulturpassagen, vorzugsweise mindestens 575 Passagen. Eine Attenuierung von Kanarienpockenvirus erfordert 200 bis 500 Kulturpassagen, bevorzugt mindestens 390 Passagen. Für jeweils 100 Passagen benötigt man je nach Pockenvirusspezies 3 bis 5 Jahre. Die Attenuierung umfaßt somit einen Zeitraum von 10 bis 20 Jahren.

Attenuierte Pockenviren, die als Pockenviruskomponente für die Paramunitätsinducer der Erfindung bevorzugt verwendet werden, sind das Hühnerpockenvirus, Stamm HP-1, 444. Passage in FHE-Kulturen, das Parapockenvirus, Stamm ORF D1701, 135. Passage in embryonalen Lammnieren-Kulturen, 37. Passage in bovinen embryonalen Lungenzellkulturen (BEL), 49. Passage in MA-Zellen (Affennieren-Zellinie), das Vaccinia-Virus, Stamm MVA (modified Vacciniavirus Ankara), 572. Passage in FHE-Kulturen und das Kanarienpockenvirus, Stamm KP-1, 535. Passage in FHE-Kulturen. Proben dieser Viren wurden bei der European Collection of Animal Cell Cultures (ECACC), PHLS Center for Applied Microbiology and Research, Portondown Salisbury, Wiltshire SP4 0JG, England, in Form von Lyophilisaten hinterlegt. Sie haben die Hinterlegungsbezeichnungen V94012709, V94012706, V94012707 bzw. V94012708 und erhalten. Es können jedoch auch andere attenuierte Pockenvirenstämme mit paramunisierenden Eigenschaften zur Herstellung der erfindungsgemäßen multipotenten Paramunitätsinducer verwendet werden.

Zur Herstellung der rekombinierten Pockenviren im Sinne der Erfindung eignen sich verschiedenen Verfahren. Nach einem bevorzugten Verfahren werden durch simultane Infektion zweier verschiedener Pockenvirenstämme mit paramunisierenden Eigenschaften Rekombinationsereignisse provoziert, bei denen Teile der für die paramunisierenden Eigenschaften kodierenden Bereiche des einen Virus in das Genom eines anderen Pockenvirus eingeführt werden. Anschließend werden rekombinierte Pockenviren, die die paramunisierenden Aktivitäten zweier verschiedener Pockenvirenstämme vereinigen, durch Plaque-Endverdünnungs-Passagen und geeignete Screeningverfahren identifiziert. Zur simultanen Infektion eignen sich beispielsweise verschiedene Kombinationen von Hühnerpockenvirus, Vaccinia MVA-, Kanarienpocken- oder Parapockenvirus.

Ein anderes Verfahren zur Herstellung rekombinierter Viren bedient sich zusätzlich gentechnologischer Techniken und des Vorgangs der homologen Rekombination. Hierzu ist es zunächst notwendig, die für die paramunisierenden Eigenschaften der Pockenviren kodierenden Bereiche zu identifizieren. Letzteres erfolgt mittels an sich bekannter Techniken.

Beispielsweise können die paramunisierenden Strukturproteine mittels biochemischer oder immunochemischer Methoden fraktioniert und auf ihre paramunisierenden Eigenschaften untersucht werden. Letzteres kann beispielsweise mittels der nachstehend genannten *in vitro*- und *in vivo*-Tests erfolgen. Nach Identifizierung der paramunisierenden Strukturproteine können durch Sequenzierung N-terminaler Sequenzen des gesamten Proteins oder dessen proteolytischer oder chemischer Spaltprodukte Aminosäuresequenzinformationen erhalten werden, die über entsprechend entworfene Oligonucleotidproben das Screenen nach den entsprechenden DNA-Abschnitten in geeigneten Genbanken erlauben, sei es mittels herkömmlicher Hybridisierungstechniken oder der Polymerase-Kettenreaktion (polymerase chain reaction; PCR). Eine weitere Möglichkeit der Identifizierung besteht darin, durch Hybridisierung unter Bedingungen verminderter Stringenz mit bekannten DNA-Proben einer Pockenvirusspezies, die für Proteine mit paramunisierenden Eigenschaften spezifisch sind, die entsprechenden homologen DNA-Abschnitte anderer Pockenviren zu isolieren, die ebenfalls für Proteine mit paramunisierender Wirkung kodieren. Auch in diesem Verfahren kann die konventionelle Methode des Screenings durch die PCR mit entsprechenden, den bekannten Sequenzen homologen Primern ersetzt werden.

DNA-Abschnitte anderer Pockenviren, die zu bekannten DNA-Abschnitten bestimmter Pockenviren homolog sind und für paramunisierende Proteine kodieren, lassen sich auch mittels Restriktionsenzym-Kartierung des Genoms verschiedener verwandter Pockenviren und Vergleich der Bereiche mit ähnlichen Restriktionsenzym-Schnittstellenmuster identifizieren. Solche vergleichende Restriktionsenzymkartierung ist beispielweise in F. Rafii and D. Burger,: Comparison of contagious Ecthyma Virus genomes by restriction endonucleases, Arch. Virol. 84, 283-289, (1985), beschrieben.

Eine weitere Methode zur Identifizierung von DNA-Abschnitten, die für paramunisierende Eigenschaften kodieren, besteht darin, für das Screening von Genbanken mono- oder polyklonale Antikörper einzusetzen, die virale Proteine mit paramunisierenden Eigenschaften erkennen. In einem solchen Verfahren wurde beispielsweise das Gen von Kaninchenpockenvirus identifiziert, das für das bei Pockenviren vorkommende 14 kD Protein (Fusionsprotein) mit paramunisierenden Eigenschaften kodiert.

Im Anschluß an die Identifizierung und Klonierung der DNA-Abschnitte, die für paramunisierende virale Proteine kodieren, kann dann die gezielte Herstellung der erfindungsgemäßen rekombinierten Pockenviren im Wege homologer Rekombination unter Verwendung geeigneter Vektoren erfolgen. Als Insertionsstelle für die Fremd-DNA eignet sich z.B. der Locus für das Thymidinkinase-Gen, da es für Pockenviren nicht essentiell ist. Das Verfahren beinhaltet Transfektion eines Transfervektors, der den in den Pockenvirus via homologer Rekombination einzuführenden heterologen, von einem anderen Pockenvirus mit paramunisierenden Eigenschaften abgeleiteten Genabschnitt trägt, in Zellen, die mit dem zu rekombinierenden Pockenvirus infiziert wurden. Vorzugsweise tragen die Transfervektoren ein Markergen zur Selektion sowie von dem zu rekombinierenden Pockenvirus abgeleitete, homologe, flankierende Sequenzen, um homologe Rekombinationsereignisse mit einer befriedigenden Rate zu ermöglichen. Anschließend werden rekombinierte Pockenviren, die die paramunisierenden Aktivitäten zweier verschiedener Pockenvirenstämme vereinigen, wiederum durch Plaque-Endverdünnungs-Passagen und geeignete Screeningverfahren, beispielsweise immunochemische Einzelplaque-Detektion mittels mono- oder polyklonaler Antikörper, identifiziert. Die inserierten DNA-Konstrukte können auch mittels Hybridisierungstechniken nachgewiesen werden, z.B. mittels der "dot blot"-Hybridisierung. Die Expression des Inserts wird direkt durch den Nachweis des betreffenden Strukturproteins mit mono- oder polyklonalen Antikörpern oder indirekt über die Immun-Paramun-Reaktion von Versuchstieren (VSV-Challenge-Test), in Zellkulturen (Zellkultur-Challenge-Test) oder durch *in vitro* Parameter (Interferoninduktion, Bildung bestimmter Interleukine, CSF, TNF etc.) nachgewiesen. Das Ergebnis ist ein rekombinantes Pockenvirus, das als Pockenviruskomponente oder als multipotenter Paramunitätsinducer im Sinne der Erfindung dienen kann.

Besonders geeignet zur Herstellung der erfindungsgemäßen rekombinierten Pockenviren sind attenuierte Pockenviren, da von ihnen bekannt ist, daß sie nicht nur unschädlich für die Umwelt sind, sondern gleichzeitig eine sehr gute paramunisierende Wirksamkeit besitzen.

Auch die attenuierten rekombinierten Pockenviren können sowohl in vermehrungsfähiger Form, als auch in inaktivierter Form zur Herstellung der multipotenten Paramunitätsinducern bzw. der pharmazeutischen Zusammensetzungen der Erfindung verwendet werden. Bevorzugt ist die Verwendung der inaktivierten rekombinierten Pockenviren.

Beispiele für vermehrungsfähige oder inaktivierte rekombinierte attenuierte Pockenviren mit paramunisierender Wirkung, die sich als Pockenviruskomponenten eignen, sind das MVA-Virus mit für paramunisierende Proteine kodierenden Genen aus Avipox- und Parapoxviren, attenuiertes Kanarienpockenvirus mit für paramunisierende Proteine kodierenden Genen aus MVA-, Hühnerpocken- und Parapockenvirus und "viceversa"-Kombinationen aus den verschiedensten, attenuierten Pockenvirusstämmen.

Die Vermehrung der als Pockenviruskomponenten für die multipotenten Paramunitätsinducer der Erfindung verwendeten vorstehend genannten Pockenviren kann sowohl in primären als auch in sekundären Zellkulturen sowie auch in permanenten Zell-Linien erfolgen. Vermehrung und Kultivierung der erfindungsgemäß verwendeten Pockenviren erfolgt nach an sich bekannten Techniken. Attenuiertes Hühnerpockenvirus kann z.B. in primären oder sekundären Fibroblasten von H̲ühner̲embryonen (FHE-Kulturen) und Kulturen von CEF-Zellen (c̲hicken e̲mbryo f̲ibroblasts; Zell-Linie) vermehrt werden. Bevorzugt ist die Vermehrung in FHE-Kulturen. Attenuiertes Parapockenvirus kann z.B. in Lammnieren- oder embryonalen Kälberlungen (b̲ovine e̲mbryo l̲ung; BEL)-Kulturen (Primäroder Sekundär-Kulturen; beispielsweise hergestellt nach der Methode von Madin S. H., P. C. Andriese, Darby, N. B., 1957: The *in vitro* cultivation of tissues of domestic and laboratory animals. Am. J. Vet. Res. 18, 932-941.), Vero- und MA-Zellen (Nierenzellinien der afrikanischen grünen Meerkatze; ATCC CCL 81 bzw. Microbiology Associated 104) vermehrt werden. Bevorzugt ist die Vermehrung in Verozellen. Das Vaccinevirus MVA kann z.B. in FHE-Kulturen oder CEF-Zellen (Zell-Linie) vermehrt werden. Bevorzugt ist die Vermehrung in FHE-Kulturen. Kanarienpockenvirus kann in FHE-Kulturen und in CEF-Zellen vermehrt werden. Bevorzugt ist die Vermehrung in FHE-Kulturen.

Im Falle von attenuiertem Hühnerpockenvirus, Vaccinevirus MVA und attenuiertem Kanarienpockenvirus ist die Vermehrung in primären oder sekundären Zellkulturen von Säugern oder Vögeln bevorzugt, die empfänglich für die genannten Viren sind. In primären oder sekundären FHE-Kulturen erfolgt die Vermehrung vorzugsweise bei 33°C bis 39°C, besonders bevorzugt bei 37°C. Die Herstellung von FHE-Zellkulturen erfolgt nach der bekannten Methode der Trypsinierung des Rumpfes und der Gliedmaßen der 10 bis 12 Tage bebrüteten Hühnerembryonen, nach Entfernung der Eingeweide und des Kopfes (Anzucht der Fibroblasten) in entsprechenden, für Zellkulturen geeigneten Behältern aus Glas oder Kunststoff, z.B. Penicillinflaschen, bei stationärer oder rotierender (Rollerkulturen) Bebrütung. Besonders bevorzugt wird nach einer Modifizierung der dem Fachmann bekannten Technik die Verwendung des gesamten Embryos, einschließlich Kopf und Eingeweide (gemischte Zellsuspension, bessere Virusernten mit stabilerer Qualität und Quantität der paramunisierenden Eigenschaften). Vorzugsweise werden die primären Kulturen nochmals durch Trypsinisieren und erneutes Ausplattieren gereinigt. Das Anzuchtmedium besteht vorzugsweise aus Minimal Essential Medium +10% Lactalbuminhydrolysat +10% BMS (Serumersatz); das Erhaltungsmedium besteht lediglich aus MEM. Die Zellkulturen werden kurz vor dem "Dichtwerden" (80 bis 100% Zellrasen), d.h. 1 bis 3 Tage nach der Aussaat, bevorzugt 20 Stunden nach der Aussaat, mit Virus infiziert und bei 33°C bis 39°C, besonders bevorzugt bei 37°C bebrütet.

Für die Animpfung von FHE-Kulturen wird als Saatvirus vorzugsweise plaque-gereinigtes Virusmaterial des betreffenden attenuierten Pockenvirusstammes verwendet. Die bevorzugte Animpfdosis beträgt $10^{7.0}$ $KID_{50}$/100 ml Medium ($KID_{50}$ = 50% kulturinfektiöse Dosis; für KID wird auch die Abkürzung TCID (tissue culture infectious dose) synonym verwendet).

Zur Gewinnung von Suspensionen der für die multipotenten Paramunitätsinducer verwendeten attenuierten Pockenviren aus Zellkulturen werden die infizierten Zellkulturen bei 33°C bis 39°C, vorzugsweise bei 37°C, bebrütet. Die Ernte des Virusmaterials erfolgt nach 1 bis 10 Tagen, bevorzugt nach 3 bis 4 Tagen, vorzugsweise wenn der Zellrasen zu 5 bis 100% zerstört ist, besonders bevorzugt in einem Status von 5 bis 60% Abkugelung (spherical, rounded cells) und 40 bis 95% Lysis. Von der Virussuspension wird eine ausreichende Menge abgenommen, um die üblichen bakteriologischen, virologischen und mykologischen Kontrollen nach den internationalen Vorschriften zur Kontrolle auf Kontaminationen vorzunehmen. Anschließend werden die Zellen *in toto* bei +4°C, -20°C bis -80°C oder bei tieferen Temperaturen gelagert, bevorzugt bei -80°C. Bis zum Abschluß der bakteriologischen, virologischen und mykologischen Kontrolluntersuchungen verbleiben die Kulturen bei dieser Temperatur. Wenn alle Kontrollen den Sicherheitsvorschriften entsprechen, werden die Virusernten aufgetaut. Dadurch werden noch intakte Zellen zerstört und damit zellgebundenes Virus freigesetzt, wodurch sich der Virustiter erhöhen kann. Bevorzugt wird die Aufschließung der Zellen durch mehrmaliges Einfrieren und Auftauen, besonders bevorzugt wird die Aufschließung durch die Behandlung mit Ultraschall. Danach wird eine erneute Sterilitätskontrolle sowie die Kontrolle des Infektiositätstiters vorgenommen.

Zur Kultivierung und Vermehrung des attenuierten Parapockenvirus wird auf die gleiche Weise vorgegangen, wie vorstehend für das attenuierte Hühnerpockenvirus, Vaccinevirus und Kanarienpockenvirus beschrieben. Der einzige Unterschied besteht darin, daß als Zellkulturen primäre oder sekundäre Lammnieren-, embryonale Kälberlungen-Zellkulturen oder verschiedene Zell-Linien, wie z.B. Vero-Zellen oder MA-Zellen (in beiden Fällen Zellstämme aus embryonalen Affennieren), verwendet werden. Besonders bevorzugt für die Herstellung von Parapockenvirussuspensionen sind Vero-Zellen.

Zur Herstellung der erfindungsgemäßen multipotenten Paramunitätsinducer können als Pockenviruskomponenten auch die abgetrennten Hüllen nichtrekombinierter oder rekombinierter Pockenviren verwendet werden sowie durch unvollständige Synthese von Viruspartikeln entstandene aberrante Formen dieser Hüllen.

Virushüllen werden vorzugsweise durch Abspaltung der Virushülle vom Viruscore (Nucleocapsid) dadurch hergestellt, daß Suspensionen von Pockenviren, die wie vorstehend beschrieben erhalten wurden, unter reduzierenden Bedingungen mit einem Detergenz inkubiert und die auf diese Weise gespaltenen Viruspartikel mittels Dichtegradientenzentrifugation in Viruscore und Virushülle getrennt werden.

Ein weiteres bevorzugtes Verfahren zur Gewinnung von paramunisierenden Virushüllen von Pockenviren oder aberranten Formen dieser Hüllen besteht in der Infektion von für die einzelnen Pockenvirusspezies nicht permissiven Zellkulturen. Ein Beispiel einer solchen Konstellation ist die Züchtung von attenuiertem ORF-Virus in Hühnerembryofibroblasten. Die jeweiligen Pokkenvirusspezies machen in den nicht permissiven Zellkulturen einen abortiven Vermehrungszyklus durch. Das heißt, in den Viroplasmazonen der infizierten Zellen kommt es nicht zum Zusammenbau von fertigen, infektionstüchtigen Viruspartikeln. Die synthetisierten, abortiven Vorstufen des Virus ("leere Hüllen", inkomplette Formen, aberrante Formen etc.) enthalten bereits die für die Paramunisierung verantwortlichen Strukturproteine. Sie werden in das Kulturmedium abgegeben bzw. können durch Lysis der infizierten Zellen freigesetzt werden. Ihre Isolierung, Reinigung und Konzentrierung erfolgt nach den vorstehend aufgeführten Verfahren.

Zur Herstellung der vorstehend genannten Virushüllen sowie der aberranten Formen von Virushüllen ist die Verwendung der vorstehend genannten Pockenviren in attenuierter Form bevorzugt. Ein bevorzugtes Verfahren zur Herstellung von Virushüllen ist nachstehend in Beispiel 6 beschrieben.

Als Pockenviruskomponente im Sinne der Erfindung können ferner virale Polypeptide dienen. Solche viralen Polypeptide sind üblicherweise virale Strukturproteine mit paramunisierenden Eigenschaften, die auf verschiedene Art und Weise hergestellt werden können, üblicherweise durch Reinigung aus Zellkulturen, die mit Pockenviren infiziert sind. Vorzugsweise handelt es sich bei diesen Zellkulturen um die vorstehend beschriebenen, wobei sowohl jene verwendet werden können, bei denen die Wirtszellen für die Pockenviren, mit denen infiziert wurde, permissiv sind, als auch jene, die für die Pockenviren nicht permissiv sind. Die Reinigung der viralen Polypeptide erfolgt nach bekannten biochemischen oder immunochemischen Methoden, beispielsweise durch die Verwendung monoklonaler Antikörper zur Immunaffinitätschromatographie. Bevorzugte Proteine, die mit diesen Verfahren gereinigt werden, sind z.B. Strukturprotein-Fraktionen von Pockenviren mit einem Molekulargewicht von 14 KDa (Fusionsprotein) bzw. 30 KDa (Adsorptionsprotein). Das Fusionsprotein stimuliert die Phagozytose, die Interferonsynthese sowie die Bildung von Interleukinen. Das Adsorptionsprotein, das allen Orthopockenspezies gemeinsam ist, stimuliert die NK-Zellen, Granulozyten sowie Makrophagen und führt über Effektorzellen zur Bildung von Interferonen und bestimmten Interleukinen.

Paramunisierende virale Polypeptide können auch direkt durch Spaltung der Pockenviruspartikel und ihre anschließende Isolierung, Fraktionierung (z.B. nach Molekulargewicht) Reinigung und Konzentrierung (z.B. mittels Dichtegradienten, Ultrazentrifugation, Chromatographie, präparativer SDS-Polyacrylamid-Gelelektrophorese oder anderer bekannter Verfahren) gewonnen werden. Ein Verfahren zur Reinigung viraler Polypeptide aus präparativen SDS-Polyacrylamidgelen ist in Beispiel 8 dargestellt.

Im Anschluß an die im Zusammenhang mit der Herstellung rekombinierter Viren erwähnten Identifizierung von Genabschnitten, die für Proteine mit paramunisierenden Eigenschaften kodieren, können die auf diese Weise klonierten und charakterisierten DNA-Abschnitte auch zur Herstellung rekombinanter viraler Polypeptide dienen. Als Expressionssysteme zu deren Herstellung eignen sich auf Plasmiden, Phagen oder Viren basierende prokaryontische oder eukaryontische Expressionsvektoren, beispielsweise solche, die zur Expression in *E. coli*, Hefen oder Säugerzellen geeignet sind. Besonders geeignet sind jene, die sich zur Expression in Säugerzellen eignen.

Durch den Einsatz bekannter gentechnologischer Methoden (Mayr, A.: Virologische Arbeitsmethoden. Band 3. Fischer Verlag 1989) werden die DNA-Abschnitte nach den bekannten Verfahren in die Phagen- oder Plasmid-Vektoren oder auf Viren basierenden Vektoren transferiert und in den geeigneten Wirtszellen exprimiert. Die exprimierten Proteine werden dann nach den bekannten Verfahren gereinigt und stellen das Ausgangsmaterial für entsprechende Kombinationen von Pockenviruskomponenten dar. Die rekombinante Expression erlaubt nicht nur die Expression eines DNA-Abschnitts, der für ein virales Polypeptid mit paramunisierenden Eigenschaften kodiert. Vielmehr können solche DNA-Abschnitte auch in einer Weise in den Expressionsvektor kloniert werden, die die Expression des resultierenden viralen Polypeptids als Fusionsprotein zur Folge hat. Als geeignete Fusionsproteinkomponenten zur Fusion mit den viralen Polypeptidanteilen kommen eine Reihe von Polypeptidsequenzen in Frage, z.B. von der bakteriellen β-Galactosidase abgeleitete Sequenzen (lacZ), wobei die DNA-Abschnitte, die für diese Komponenten kodieren, üblicherweise in den zur Expression verwendeten Vektorsystemen bereits vorhanden sind. Die als Pockenvirenkomponenten in Sinne der vorliegenden Erfindung geeigneten Fusionsproteine können jedoch auch Fusionen aus zwei oder mehreren verschiedenen viralen Polypeptiden sein, die sich von verschiedenen paramunisierenden Pockenvirusstämmen ableiten. Letztere Fusionsproteine vereinigen paramunisierende Polypeptidbereiche verschiedener Pockenviren in einem Polypeptid und werden erhalten, indem zwei oder mehrere der DNA-Abschnitte, die für diese Polypeptidbereiche kodieren, in geeigneter Reihenfolge und im richtigen Leseraster miteinander kombiniert werden.

Für die Verwendung der Pockenviruskomponenten als multipotente Paramunitätsinducer in der Humanmedizin können weitere Reinigungsverfahren erforderlich sein. Beispielsweise kann das Präparat durch weitere an sich bekannte Reinigungsverfahren, wie z.B. Ultrazentrifugation, Dichtegradientenzentrifugation usw. weiter gereinigt werden.

Die Inaktivierung der Pockenvirusstämme bzw. der rekombinanten Virusstämme kann durch verschiedene Behandlungen erfolgen, z.B. Hitzebehandlung, auf physikalischem oder chemischem Wege oder durch pH-Einwirkung. Vorzugsweise erfolgt die Inaktivierung durch γ-Bestrahlung oder Behandlung mit β-Propiolacton, wobei die Behandlungen unter solchen Bedingungen durchgeführt werden, die die paramunisierenden Eigenschaften nicht zerstören.

In der besonders bevorzugten Ausführungsform wird die Inaktivierung des attenuierten Pockenvirus mit β-Propiolacton in einem pH-Bereich von 8 bis 9 vorgenommen. Die Behandlung mit β-Propiolacton wird vorzugsweise unter folgenden Bedingungen durchgeführt: Der pH-Wert der Virussuspension wird mit $NaHCO_3$ (8.8%-ig in Aqua dest.) auf 8.6 eingestellt. β-Propiolacton wird 1:10 mit Aqua dest. vorverdünnt und unter entsprechenden Vorsichtsmaßnahmen in einem Verhältnis von 0.5 zu 100 zur Virussuspension gegeben. Das entspricht einer Verdünnung von 0.05% β-Propiolacton. Diese Suspension wird 1 Stunde bei Kühlschranktemperatur (+4 bis +8°C) und 4 Stunden bei 37°C gerührt und anschließend ca. 12 bis 18 Stunden (über Nacht) im Kühlschrank gelagert. Die inaktivierte Suspension wird durch Zentrifugation bei 2600 U/min. über 10 Minuten gereinigt. Danach wird der pH-Wert nochmals überprüft und gegebenenfalls reguliert.

Der Nachweis der Inaktivierung erfolgt durch Verimpfung ausreichender Stichproben-Volumina des behandelten Materials in FHE-Kulturen. Wenn sich spätestens 9 Tage nach der Verimpfung keine typischen cytopathischen Veränderungen entwickelt haben, wird dies als Beweis für eine vollständige Inaktivierung gewertet. Bei Verdacht auf nicht inaktiviertes Restvirus werden 3 Subpassagen durchgeführt. Eine erneute Kontrolle auf Verunreinigung mit Viren, Bakterien oder Pilzen wird zusätzlich in geeigneten Kulturen bzw. auf geeigneten Nährböden sowie durch elektronenmikroskopische Untersuchung durchgeführt.

Vorzugsweise werden die Lösungen bzw. Suspensionen der Pockenviruskomponenten nach Aufreinigung lyophilisiert. Vor der Lyophilisierung werden den Pockenviruskomponenten vorzugsweise geeignete Stabilisatoren zugesetzt. Im Falle der Suspensionen attenuierter Pockenviren wird der Pockenvirussuspension ein Stabilisator, wie z.B. Kollidon[R], Molico[R] (Magermilchpulver) oder Gelatine zugesetzt. In der besonders bevorzugten Ausführungsform wird succinylierte Gelatine (Fa. Hausmann, St. Gallen/Schweiz) in einer Endkonzentration von 2.5% zugesetzt. Vor der Zugabe der Gelatine (50%ig) muß ihr pH-Wert auf 8.0 eingestellt werden. Dies gilt auch für Stabilisatoren anderer Art.

Das fertige Präparat wird portioniert, z.B. in Mengen von 2 ml, und lyophilisiert. Der lyophilisierte multipotente Paramunitätsinducer bleibt bei einer Lagerung zwischen +4°C und +8°C oder niedrigeren Temperaturen mindestens 10 Jahre stabil. Die im VSV-Challenge-Test oder im Zellkultur-Challenge-Test festgestellten Wirkungseinheiten des Lyophilisates verändern sich unter diesen Bedingungen in einem Zeitraum von mindestens 10 Jahren nicht (der Begriff Wirkungseinheit ist nachstehend definiert). Vor der endgültigen Lagerung wird das Lyophilisat nochmals bakteriologisch, virologisch und toxikologisch auf Reinheit überprüft.

Die auf die oben beschriebene Weise hergestellten Pockenviruskomponenten zur Herstellung der erfindungsgemäßen Paramunitätsinducer werden zur Potenzierung ihrer Wirksamkeit in verschiedenen Mischungsverhältnissen miteinander kombiniert. Im Falle attenuierter Pockenviren beträgt das Mischungsverhältnis vorzugsweise 1:1. Es sind auch Kombinationen z.B. in folgenden Mischungsverhältnissen möglich:

1. Hühnerpockenvirus- und Parapockenvirus 5:1 bis 1:5, vorzugsweise 2:1 bis 1:2;
2. Hühnerpockenvirus-, Parapockenvirus- und Vacciniavirus MVA 6:3:1 bis 1:3:6, vorzugsweise 6:3:1, besonders bevorzugt 1:1:1;
3. Hühnerpockenvirus-, Parapockenvirus-, MVA- und Kanarienpockenvirusinducer 1:1:1:1.

Entsprechende Mischungsverhältnisse können auch für die anderen erfindungsgemäßen Pockenviruskomponenten verwendet werden. Andere Mischungsverhältnisse sind jedoch ebenfalls zur Herstellung der multipotenten Paramunitätsinducer der Erfindung geeignet.

Die Kombinierung der Pockenviruskomponenten zur Herstellung der multipotenten Paramunitätsinducer der Erfindung kann zu verschiedenen Zeitpunkten innerhalb des Herstellungsverfahrens erfolgen, vorzugsweise vor der Behandlung zur Inaktivierung, vor dem Lyophilisieren oder nach dem Lyophilisieren. Bei Verwendung von Pockenviren als Pockenviruskomponenten werden die Komponenten vorzugsweise vor der Inaktivierungsbehandlung kombiniert.

Die Wirksamkeit sowohl der erfindungsgemäßen multipotenten Paramunitätsinducer als auch der einzelnen Pockenviruskomponenten kann mittels verschiedener Verfahren nachgewiesen werden:
Als *in vivo*-Nachweis wird das VSV-Infektionsmodell in der Babymaus verwendet; vgl. Mayr et al. (1986), a.a.O. Es kann gleichzeitig zur Bestimmung der Wirkungseinheiten (WE/ml) dienen. In der Tabelle 1 ist die Wirksamkeit verschiedener Verdünnungen von PIND-AVI und PIND-ORF im VSV-Modell dargestellt. Der Wirkungsindex eines Präparates bzw. einer Verdünnungsstufe wird nach der folgenden Formel berechnet:

$$\text{Wirkungsindex (WI)} = \frac{b\text{-}a}{b} \times 100$$

a = % Mortalität der Versuchsgruppe
b = % Mortalität der Kontrollgruppe

Diejenige Verdünnungsstufe, bei der noch ein Wirkungsindex von mindestens 20 erzielt wird, enthält 1 WE pro 0,1 ml (Infektionsdosis pro Babymaus) bzw. 10 WE/ml. Im Beispiel mit PIND-AVI liegt der Endtiter bei 1:16; die Charge enthält demnach 160 WE/ml).

Als *in vitro*-Nachweis der Wirksamkeit sowohl der erfindungsgemäßen multipotenten Paramunitätsinducer als auch der einzelnen Pockenviruskomponenten können verschiedene Test-Verfahren eingesetzt werden: a) die Aktivität peritonealer NK-Zellen 24 Stunden nach Behandlung mit PIND-AVI im 4 Stunden-Chromium 51-Freisetzungstest; Mayr et al. 1986, a.a.O., b) die koloniestimulierende Aktivität im Serum von Mäusen 8 Stunden nach Behandlung; vgl. Wolf, G., 1986: Koloniestimulierende Aktivität in Mäuseseren nach Vorbehandlung mit Inducern aus Pockenviren und anderen Mikroorganismen; Czerny 1990, a.a.O., und c) die Interferonsynthese in mononukleären Leukozyten im peripheren Blut; vgl. Steinmaßl, M. und G. Wolf, 1990: Bildung von Interleukin-2 und Interferon$_a$ durch mononukleäre Leukozyten des Schweines nach *in vitro*-Stimulation mit verschiedenen Viruspräparaten, J. Vet. Med. B 37, 321-331. Es können aber auch andere Nachweismethoden, wie z.B. die Messung der Aktivität des Tumornekrose-Faktor (TNF) in vorbehandelten Kaninchen oder Mäusen im L929-der Challenge-Test in der adulten Maus mit dem Aujeszkyvirus, die Bestimmung der koloniestimulierenden Aktivität (CSA) im Serum verschiedener Tiere und die Messung der Granulozytenaktivität bei Mensch und Tier im "FACS-Analyser" (Partikelaufnahme, "respiratory burst") eingesetzt werden.

Die mittels der vorstehend beschriebenen Verfahren erhaltenen multipotenten Paramunitätsinducer können als Ausgangsprodukt zur Herstellung pharmazeutischer Zusammensetzungen zur Verwendung als Arzneimittel dienen. Zu diesem Zweck kann das Lyophilisat entsprechend dem Ausgangsvolumen mit üblichen pharmazeutischen Trägern, vorzugsweise mit sterilem, pyrogenfreien Aqua dest. versetzt werden. In dieser Zubereitungsform eignet es sich sowohl für die parenterale (z.B. intramuskulär, subcutan) als auch für die lokale Verabreichung. Die erfindungsgemäßen multipotenten Paramunitätsinducer sind atoxisch, pyrogenfrei und verursachen beispielsweise bei intramuskulärer Injektion keine abnormen postvaccinalen Reaktionen.

Die erfindungsgemäßen pharmazeutischen Zusammensetzungen umfassen auch andere Zubereitungsformen. Beispiele für Zubereitungsformen zur lokalen Applikation sind Lutschtabletten, Salben, Zäpfchen, Nasentropfen, Inhalationssprays etc. Beispiele für Zubereitungsformen zur parenteralen Applikation sind Injektionspräparate. Sie werden nach an sich bekannten Verfahren hergestellt.

Weitere geeignete Zubereitungsformen der pharmazeutischen Zusammensetzung im Sinne der Erfindung sind beispielsweise Rektal- oder Vaginalzäpfchen. In der Form der beiden letztgenannten Zubereitungsformen eignen sie sich zur Behandlung von Hämorrhiden bzw. Entzündungen oder anderen Schädigungen der Vagina. Bei der Konfektionierung muß darauf geachtet werden, daß nicht Sterilität und Haltbarkeit durch Bestandteile des Trägers, beispielsweise solche mit proteolytischer Aktivität, beeinträchtigt werden.

In der Prophylaxe werden die multipotenten Paramunitätsinducer vorzugsweise 1 bis 3 mal im Abstand von 24 Stunden verabreicht. Beim therapeutischen Einsatz werden je nach Schwere der Erkrankungen vorzugsweise 1 bis 3 Behandlungen pro Tag über 3 bis 5 Tage bzw. bis zum Abklingen der klinischen Symptome durchgeführt. Die Dosis richtet sich nach den zu behandelnden Symptomen und der gewählten Applikationsform. Sie beträgt bei parenteraler Applikation üblicherweise 2 ml, wobei eine Dosis vorzugsweise mindestens 160 Wirkungseinheiten enthält. Dosis und Applikationsform können jedoch im Einzelfall in geeigneter Weise abgewandelt werden.

Die folgenden Beispiele dienen zur weiteren Erläuterung der Erfindung.

**Beispiel 1**

Als Ausgangsmaterial zur Herstellung einer erfindungsgemäßen Pockenviruskomponente diente der Hühnerpockenstamm HP1, der aus einem klinisch generalisiert kranken Huhn isoliert und über 440 Kulturpassagen in Hühnerembryofibroblasten (FHE)-Kulturen attenuiert worden war. Im Verlauf der Attenuierungspassagen waren in regelmäßigen Abständen Klonselektionen mittels Plaque-Endverdünnungs-Technik sowie Kontrolluntersuchungen auf Immunogenität und paramunisierende Wirksamkeit eingeschaltet worden. Zur Herstellung der Pockenviruskomponente der Erfindung diente die 440. FHE-Passage, die in den letzten 3 Passagen nochmals mittels Plaquetechnik einer Klonselektion unterzogen worden war, um mit genetisch einheitlichem Material arbeiten zu können. Die Unterschiede zwischen Virusmaterial aus niedrigen und hohen Zellkulturpassagen in bezug auf die paramunisierende Wirksamkeit sind in Tabelle 2 gegenübergestellt.

FHE-Kulturen wurden nach einer modifizierten Methode der allgemein üblichen Technik durch Anzüchtung des Hühnerembryos in toto (einschließlich Kopf und Innereien) hergestellt; vgl. Mayr A., P. A. Bachmann, B. Bibrack und G. Wittmann, 1974: Virologische Arbeitsmethoden, Band 1, VEB Gustav Fischer Verlag Jena. In Änderung der üblichen Technik wurde für die Anzucht der Zellen ein vollsynthetisches Medium aus 10% BMS (Serumersatzmedium), 10% Lactalbuminhydrolysat und MEM (minimal essential medium) verwendet. Die ca. 90% dichten Monolayer-Zellkulturen wurden mit dem Virusstamm HP1, 440. Passage, Infektiositätstiter $10^{7.5}$ KID$_{50}$/ml, beimpft. Die Infektionsdosis betrug $10^7$ KID$_{50}$/ 100 ml. Als Erhaltungsmedium diente MEM (ohne Antibiotika). Die Virusernte erfolgte zu einem Zeitpunkt, bei dem der Zellrasen zu 5 bis 100% virusspezifisch verändert war. Noch vorhandener Zellrasen wurde durch Schütteln der Kulturgefäße abgelöst. Um noch intakte Zellen aufzuschließen, wurde das zellhaltige Virusmedium mit Ultraschall behandelt (Branson Sonifier 40-50 Watt, 3 Minuten).

Zur Kontrolle der Sterilität und Infektiosität wurden der Virusernte Proben entnommen. Nur Virusernten, die frei von Kontaminationen (Bakterien, Viren, Pilze, Mykoplasmen) waren und einen Infektiositätstiter von mindestens $10^{7.25}$/ml besaßen, wurden weiterverarbeitet. Virusernten können bis zur weiteren Verarbeitung bei +4 oder -80°C gelagert werden.

Vor Beginn der Inaktivierung mit β-Propiolacton wurde der pH-Wert des Virusmaterials auf einen Wert von 8.6 eingestellt. β-Propiolacton wurde in einer Konzentration von 0.05% zugesetzt. Anschließend wurde das Reaktionsgemisch 1 Stunde im Kühlschrank und anschließend 4 Stunden im Brutschrank (37°C) unter Rühren inaktiviert. Der Inaktivierungsvorgang wurde durch stationäre Lagerung der Virussuspension im Kühlschrank über Nacht abgeschlossen. Die inaktivierte Virussuspension wurde durch Zentrifugation bei 2600 U/min. grob gereinigt. Der so gewonnene Inducer wurde als PIND-AVI bezeichnet und bei Temperaturen von +4°C oder -60°C gelagert und nach Abschluß der Kontroll-Untersuchungen lyophilisiert.

Die Prüfung der erhaltenen attenuierten Pockenvirus-Suspensionen auf Spezifität, Reinheit und Unschädlichkeit erfüllte die Forderungen des Europäischen Arzneibuches in folgenden Kriterien:

1. Elektronenmikroskopische Kontrolle mit den üblichen Methoden zeigten im Präparat typische, z.T. leere Pockenviruspartikel. Das Präparat war frei von anderen mikrobiellen Strukturen.

2. Die üblichen Kontrollen auf mikrobielle Kontamination bewiesen, daß das Präparat frei von anderen Viren, Bakterien, Pilzen und Mykoplasmen war.

3. Nach den Richtlinien des Europäischen Arzneibuches wurden Toxizität, Teratogenität, Pyrogenität geprüft und keine positiven Ergebnisse festgestellt.

4. Kontrolle auf den Gehalt an nicht inaktiviertem Restvirus: hierfür wurde mit dem fertigen Pockenvirus-Präparat in üblicher Weise eine Titrierung in FHE-Kulturen durchgeführt. In weiteren 3 Folgepassagen lag der Restvirusgehalt bei 0.

Die paramunisierende Wirkung der Pockenviruskomponente PIND-AVI wurde im VSV-Infektionsmodell nachgewiesen. Die Ergebnisse sind in Tabelle I dargestellt.

**Beispiel 2**

In analoger Weise wie in Beispiel 1 beschrieben wurde eine erfindungsgemäße Pockenviruskomponente aus dem attenuierten Vaccinia-Virus MVA in FHE-Kulturen hergestellt. Wie aus der Tabelle 2 zu ersehen ist, verbesserte sich die paramunisierende Aktivität des Virusstammes durch eine Attenuierung über mindestens 500 Kulturpassagen. Die Prüfungen auf Reinheit, Unschädlichkeit, Spezifität und Wirksamkeit erfolgten wie in Beispiel 1 beschrieben. Es wurden die gleichen Ergebnisse wie in Beispiel 1 erzielt. In Tabelle 2 sind die Ergebnisse dargestellt.

**Beispiel 3**

In analoger Weise wie in Beispiel 1 beschrieben wurde eine erfindungsgemäße Pockenviruskomponente aus dem attenuierten Kanarienpockenvirus, Stamm KP1, in FHE-Kulturen hergestellt. Wie aus der Tabelle 2 zu ersehen ist, verbesserte sich die paramunisierende Aktivität des Virusstammes durch eine Attenuierung über mindestens 390 Kulturpassagen. Die Prüfungen auf Reinheit, Unschädlichkeit, Spezifität und Wirksamkeit erfolgten wie in Beispiel 1 beschrieben. Es werden die gleichen Ergebnisse wie in Beispiel 1 erzielt. Die Ergebnisse sind in Tabelle 2 beispielhaft dargestellt.

**Beispiel 4**

In analoger Weise wie in Beispiel 1 beschrieben wurde eine erfindungsgemäße Pockenviruskomponente aus dem attenuierten Parapockenvirus, Stamm D 1701, in MA-oder Vero-Zellen (permanente Zell-Linie aus Affennieren) hergestellt. Wie aus der Tabelle 2 zu ersehen ist, verbesserte sich die paramunisierende Aktivität des Virusstammes durch eine Attenuierung über mindestens 170 Kulturpassagen. Die Prüfungen auf Reinheit, Unschädlichkeit, Spezifität und Wirksamkeit erfolgen wie in Beispiel 1 beschrieben. Es wurden die gleichen Ergebnisse wie in Beispiel 1 erzielt.

In den Tabellen 1 und 2 sind die Ergebnisse für die Einzelkomponenten (Produkte der Beispiele 1 bis 4) dargestellt.

**Beispiel 5**

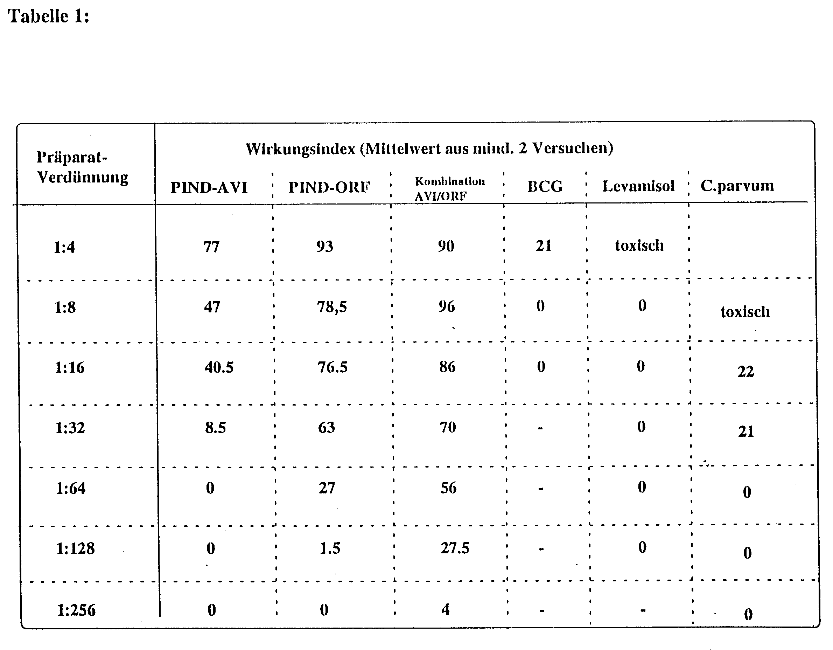

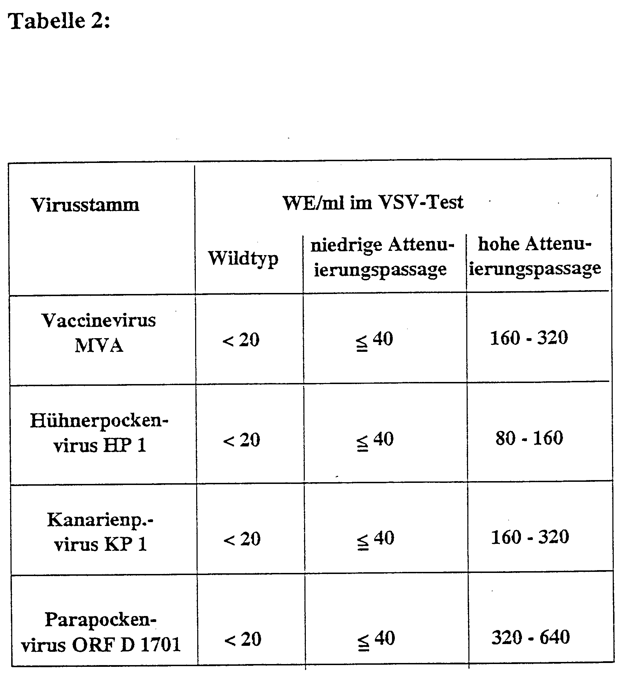

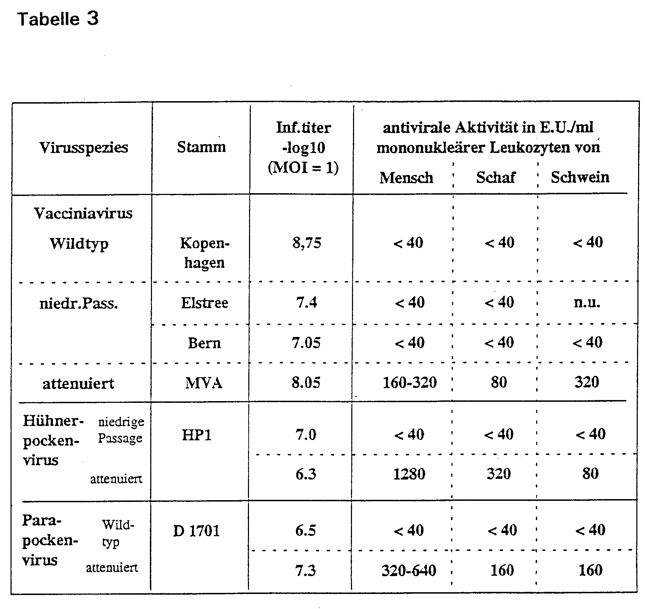

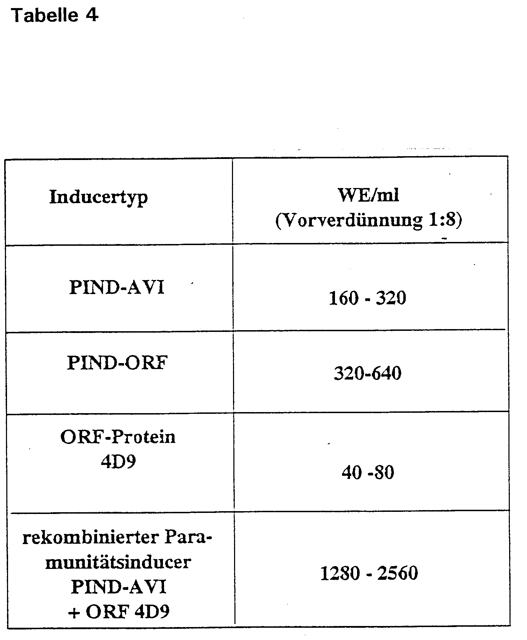

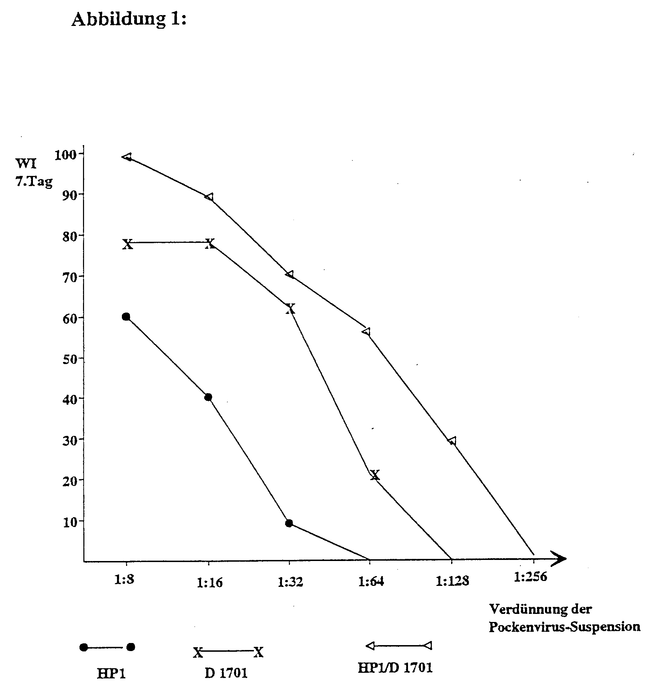

Die Herstellung eines multipotenten Paramunitätsinducers im Sinne der Erfindung wurde durch die Mischung von je 2 der in Beispiel 1 bis 4 beschriebenen attenuierten, inaktivierten Pockenvirussuspensionen in einem Mengenverhältnis von 1:1 in einem Präparat bewirkt. Hergestellt wurden eine Mischung der Pockenviruskomponenten PIND-AVI und PIND-ORF sowie der Pockenviruskomponenten Hühnerpockenvirus HP1 und Parapockenvirus ORF D 1701. Wie aus der Tabelle 1 und Abbildung 1 zu ersehen ist, potenziert sich durch die Kombination der Inducer die paramunisierende Wirksamkeit. (Bei einer additiven Wirkung wären sowohl die Wirkungsindizes der einzelnen Verdünnungen, als auch die WE/ml unverändert. Die Erhöhung beider Bezugsgrößen im dargestellten um bis 20-40% (Wirkungsindex der Kombination im Vergleich zur 1:8 Verdünnung von D 1701 bzw. HP1) bzw. um 200-400% (WE/ml der Kombination im Vergleich zu D 1701 bzw. HP1) beweist dies eindeutig. Die Prüfungen auf Reinheit, Unschädlichkeit, Spezifität und Wirksamkeit erfolgten gemäß Beispiel 1. Es wurden die gleichen Ergebnisse wie in Beispiel 1, mit Ausnahme einer erhöhten paraspezifischen Aktivität, erzielt.

**Beispiel 6**

Vacciniavirus, Hühnerpockenvirus HP1 und Parapockenvirus ORF D1701 wurden analog Beispiel 1 attenuiert und die Wirkung der Attenuation auf die paramunisierende Wirkung in einem Test verglichen, bei dem die Induktion der Interferonsynthese bei mononukleären Leukozyten des peripheren Blutes gemessen wird. Die Ergebnisse sind in Tabelle 3 dargestellt.

**Beispiel 7:**

Zur Herstellung einer erfindungsgemäßen Pockenviruskomponente wurden Suspensionen von Vaccinia-Virus MVA und Parapockenvirus ORF D1701 mit PBS auf einen Proteingehalt von 2 mg/ml eingestellt und mit 1% Nonidet P-40 (NP-40; Sigma) und 50 $\mu$l 2-Mercap-toethanol für 60 min bei 37°C inkubiert. Nach Unterschichtung mit 3,5 ml eines 36%-igen Sucrosekissens (w/v, in PBS) und Zentrifugation für 45 min in einer Ultrazentrifuge in einem Beckmann SW-60 Ti-Rotor bei 40.000 Upm (230.000 g) und 4°C wurde ein Pellet erhalten, das die Viruscores repräsentierte. Die abgetrennten Virushüllen befanden sich im Überstand. Der Überstand wurde ausgiebig gegen PBS dialysiert und dann zur Herstellung der erfindungsgemäßen multipotenten Paramunitätsinducer verwendet.

**Beispiel 8**

Zur Isolierung einzelner Virusproteinbanden wurden 12%-ige SDS-Polyacrylamidgele vorbereitet. Die verwendeten Puffer und Lösungen waren wie bei Laemmli U. K., (1970), Cleavage of structural proteins during the assembly of the head of bacteriophage T4, Nature 227, 680-685, und Mayr, A., Bachmann, P. A., Bibrack, B., Wittmann, G., 1989, Virologische Arbeitsmethoden, Teil III: Biochemische und biophysikalische Methoden, Gustav Fischer Verlag, Stuttgart, beschrieben. Suspensionen von Vacciniavirus MVA und Parapockenvirus ORF D1701 wurden in 50 $\mu$l Probenpuffer aufgekocht. Pro Geltasche wurden je 50 $\mu$l der Proben

aufgetragen, was einer Virusmenge von 10 µg/Geltasche entsprach. Anschließend wurden die Virusproteine bei 50 V und 20-30 mA für ca. 16 Stunden elektrophoretisch aufgetrennt. Zur Identifizierung der Proteine wurden zwei Molekulargewichtsstandards mitgeführt (MW SDS-70L Kit und MW SDS-200 Kit; Sigma) die einen Bereich von 14.300 bis 205.000 Dalton abdeckten. Nach der Auftrennung wurden die Spuren (lanes) mit den Markerproteinen und eine, auf der virale Proteine aufgetrennt worden waren, abgetrennt und zur Lokalisierung der Markerbanden einer Silberfärbung unterzogen. Der Rest des Proteingels wurde in der Zwischenzeit bei 4°C in Elektrodenpuffer aufbewahrt. Mit dem silbergefärbten Gelstreifen als Orientierungshilfe wurden anschließend aus dem Molekulargewichtsbereich um 14 kD alle Proteinbanden mit einem Skalpell ausgeschnitten. Jeweils sechs solcher Gelstückchen (ca. 0,5 ml Volumen) wurden dann der Reihe nach in dafür vorgesehene Vertiefungen eines Extraphor Electrophoretic Concentrator (Pharmacia LKB) gebracht. Die einzelnen Proteinbanden wurden über 30 min bei Raumtemperatur und 100 V aus den Gelstückchen eluiert und gleichzeitig in dem "Hochsalz-Puffer" gefällt. Die gefällten Proteine wurden abgenommen, vereinigt und 30 min bei 10.000 Upm zentrifugiert. Nach Resuspension des Sediments in 1 ml PBS und Dialyse über Nacht bei 4°C in Dialyseschläuchen eines Ausschlußmolekulargewichts von 8 kD wurden die Präparationen für 30 min bei 10.000 Upm steril zentrifugiert und zur Herstellung der multipotenten Paramunitätsinducer wie vorstehend beschrieben weiterverarbeitet.

**Beispiel 9**

Verschiedene Pockenviruskomponenten wurden im VSV-Test einem Vergleich in bezug auf ihre paramunisierenden Eigenschaften unterzogen. Dabei wurden die Pockenviruskomponenten PIND-AVI, PIND-ORF und das aus Zellkulturen über Immunaffinitätschromatographie gereinigte ORF-Protein 4D9 mit dem rekombinierten Pockenvirus PIND-AVI+ORF 4D9 verglichen, in das durch homologe Rekombination der DNA-Abschnitt eingeführt worden war, der für das ORF 4D9-Polypeptid kodiert. Wie aus Tabelle 4 zu entnehmen ist, war die Wirkung des multipotenten Paramunitätsinducers deutlich gegenüber der der Einzelkomponenten gesteigert.

**Beispiel 10**

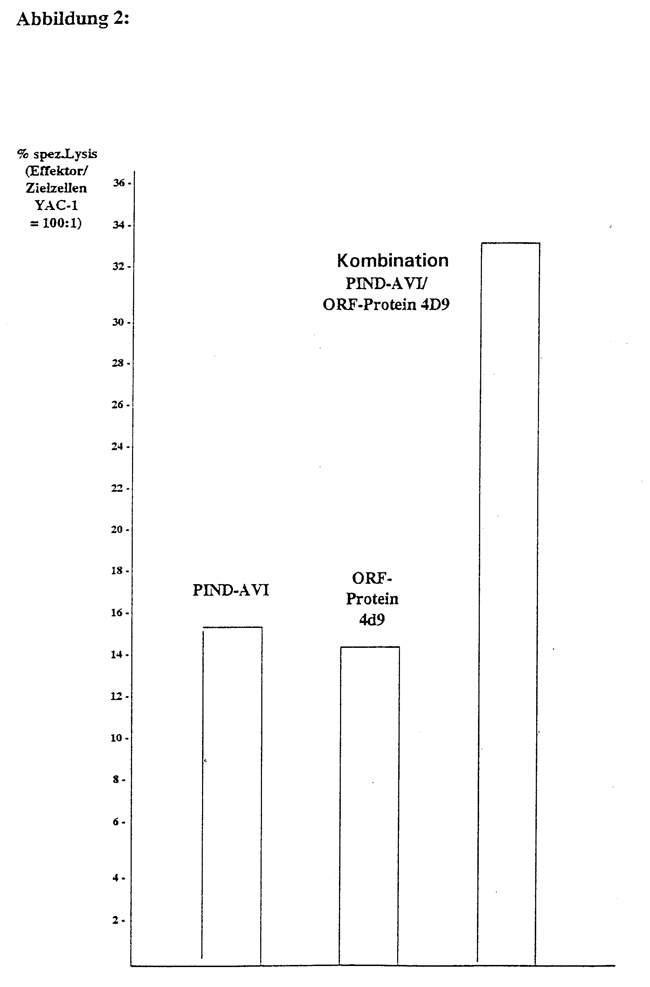

Die potenzierende Wirkung der kombinierten multipotenten Paramunitätsinducer im Vergleich zur Wirkung der Einzelkomponenten wurde im Chromium 51-Freisetzungstest nachgewiesen; vergl. Mayr. et al. 1986 a.a.O. Verglichen wurden die Pockenviruskomponenten PIND-AVI und das ORF-Protein-4D9, das aus Zellkulturüberständen von Zellen aufgereinigt worden war, die zuvor mit Parapockenvirus ORF D1701 infiziert worden waren, mit einer Kombination aus PIND-AVI und ORF-Protein-4D9. Die Ergebnisse sind in Abbildung 2 dargestellt.

**Patentansprüche**

1. Paramunitätsinducer auf der Basis von Pockenviruskomponenten, dadurch gekennzeichnet, daß der Paramunitätsinducer eine Kombination zweier oder mehrerer Pockenviruskomponenten umfaßt, die sich von unterschiedlichen Pockenvirusstämmen mit paramunisierenden Eigenschaften ableiten.

2. Paramunitätsinducer nach Anspruch 1, dadurch gekennzeichnet, daß die Pockenviruskomponenten Pockenviren sind.

3. Paramunitätsinducer nach Anspruch 1, dadurch gekennzeichnet, daß eine oder mehrere der Pockenviruskomponenten Hühnerpockenvirus HP-1, Parapockenvirus ORF D1701, Vaccinia-Virus MVA, oder Kanarienpockenvirus KP-1 mit den ECACC Hinterlegungsbezeichnungen V94012709, V94012706, V94012707 bzw. V94012708 sind.

4. Paramunitätsinducer nach Anspruch 1, dadurch gekennzeichnet, daß die Pockenviruskomponenten Virushüllen oder aberrante Formen von Virushüllen sind, die aus Pockenviren gewonnen wurden.

5. Paramunitätsinducer nach Anspruch 2 oder 3, dadurch gekennzeichnet, daß die Pockenviren inaktiviert sind.

6. Paramunitätsinducer nach Anspruch 5, dadurch gekennzeichnet, daß die Inaktivierung der Pockenviren durch Behandlung mit β-Propiolacton in einem pH-Bereich von 8 bis 9 erfolgt.

7. Paramunitätsinducer nach Anspruch 1, dadurch gekennzeichnet, daß die Pockenviruskomponenten einzelne virale Proteine sind, die durch biochemische oder immunochemische Methoden von Kulturen gewonnen wurden, die mit Pockenviren infiziert worden waren.

8. Paramunitätsinducer nach Anspruch 1, dadurch gekennzeichnet, daß die Pockenviruskomponenten durch prokaryontische oder eukaryontische Expression gewonnene rekombinante virale Polypeptide sind, die sich wenigstens teilweise von einem oder mehreren der viralen Polypeptide von Pockenviren ableiten.

9. Paramunitätsinducer nach einem der Ansprüche 2, 4, 5, 6, 7 oder 8, dadurch gekennzeichnet, daß die Pockenviren frisch isoliert sind.

10. Paramunitätsinducer nach einem der Ansprüche 2, 4, 5, 6, 7 oder 8, dadurch gekennzeichnet, daß die Pockenviren attenuiert sind.

11. Paramunitätsinducer nach Anspruch 9 oder 10, dadurch gekennzeichnet, daß die Pockenviren rekombiniert sind.

12. Verfahren zur Herstellung von Paramunitätsinducern, dadurch gekennzeichnet, daß man zwei oder mehrere Pockenviruskomponenten kombiniert, die sich von unterschiedlichen Pockenvirusstämmen mit paramunisierenden Eigenschaften ableiten.

13. Verwendung von Paramunitätsinducern auf der Basis von Kombinationen von Pockenviruskomponenten, die sich von unterschiedlichen Pockenvirusstämmen ableiten, zur Herstellung von pharmazeutischen Zusammensetzungen.

## Claims

1. Paramunity inducers based on poxvirus components, characterized in that the paramunity inducer comprises a combination of two or more poxvirus components derived from different poxvirus strains with paramunizing properties.

2. Paramunity inducers according to claim 1, characterized in that the poxvirus components are poxviruses.

3. Paramunity inducers according to claim 1, characterized in that one or more of the poxvirus components are fowlpox virus HP-1, parapox virus ORF D1701, vaccinia virus MVA, or canarypox virus KP- 1, respectively identified by the ECACC deposition numbers V94012709, V94012706, V94012707 and V94012708.

4. Paramunity inducers according to claim 1, characterized in that the poxvirus components are virus envelopes or aberrant forms of virus envelopes that have been recovered from poxviruses.

5. Paramunity inducers according to claim 2 or claim 3, characterized in that the poxviruses have been inactivated.

6. Paramunity inducers according to claim 5, characterized in that the poxviruses are inactivated by treating them with β-propiolactone in a pH range of 8 to 9.

7. Paramunity inducers according to claim 1, characterized in that the poxvirus components are individual viral proteins recovered by biochemical or immunochemical methods from cultures that had been infected with poxviruses.

8. Paramunity inducers according to claim 1, characterized in that the poxvirus components are recombinant viral polypeptides recovered by prokaryotic or eukaryotic expression, at least some of which are derived from one or more of the viral polypeptides of poxviruses.

9. Paramunity inducers according to any of claims 2, 4, 5, 6, 7 or 8, characterized in that the poxviruses are freshly isolated.

10. Paramunity inducers according to any of claims 2, 4, 5, 6, 7 or 8, characterized in that the poxviruses are attenuated.

11. Paramunity inducers according to claim 9 or 10, characterized in that the poxviruses are recombinant.

12. Method for the preparation of paramunity inducers, characterized by combining two or more poxvirus components that are derived from different poxvirus strains with paramunizing properties.

13. Use of paramunity inducers based on combinations of poxvirus components derived from different poxvirus strains for the preparation of pharmaceutical compositions.

## Revendications

1. Inducteurs de paramunité à base de composants de poxvirus, caractérisés en ce que l'inducteur de paramunité comprend une combinaison de deux ou plusieurs composants de poxvirus dérivés de différentes souches de poxvirus ayant des propriétés paramunisantes.

2. Inducteurs de paramunité selon la revendication 1, caractérisés en ce que les composants de poxvirus sont des poxvirus.

3. Inducteurs de paramunité selon la revendication 1, caractérisés en ce qu'un ou plusieurs des composants du poxvirus sont du poxvirus de poule HP-1, du parapoxvirus ORF D1701, du virus de la vaccine MVA ou du poxvirus de canari KP-1 ayant les numéros de dépôt auprès de l'ECACC respectivement V94012709, V94012706, V94012707 et V94012708.

4. Inducteurs de paramunité selon la revendication 1, caractérisés en ce que les composants du poxvirus sont des enveloppes virales ou des formes aberrantes d'enveloppes virales, qui ont été obtenues à partir de poxvirus.

5. Inducteurs de paramunité selon l'une des revendications 2 ou 3, caractérisés en ce que les poxvirus sont inactivés.

6. Inducteurs de paramunité selon la revendication 5, caractérisés en ce que l'inactivation des poxvirus s'effectue par traitement avec de la β-propiolactone dans un domaine de pH de 8 à 9.

7. Inducteurs de paramunité selon la revendication 1, caractérisés en ce que les composants du poxvirus sont des protéines virales individuelles, qui ont été recueillies par des méthodes biochimiques ou immunochimiques à partir de cultures qui avaient été infectées avec des poxvirus.

8. Inducteurs de paramunité selon la revendication 1, caractérisés en ce que les composants du poxvirus sont des polypeptides viraux recombinants obtenus par expression procaryote ou eucaryote, qui dérivent au moins partiellement d'un ou plusieurs des polypeptides viraux de poxvirus.

9. Inducteurs de paramunité selon l'une quelconque des revendications 2, 4, 5, 6, 7 ou 8, caractérisés en ce que les poxvirus sont fraîchement isolés.

10. Inducteurs de paramunité selon l'une quelconque des revendications 2, 4, 5, 6, 7 ou 8, caractérisés en ce que les poxvirus sont atténués.

11. Inducteurs de paramunité selon l'une des revendications 9 ou 10, caractérisés en ce que les poxvirus sont recombinés.

12. Procédé pour la préparation d'inducteurs de paramunité, caractérisé en ce qu'on combine deux ou plusieurs composants de poxvirus qui proviennent de souches de poxvirus différentes ayant des propriétés paramunisantes.

13. Utilisation d'inducteurs de paramunité à base de combinaisons de composants de poxvirus qui proviennent de souches de poxvirus différentes pour la préparation de compositions pharmaceutiques.

## Tabelle 1:

| Präparat-Verdünnung | Wirkungsindex (Mittelwert aus mind. 2 Versuchen) | | | | | |
|---|---|---|---|---|---|---|
| | PIND-AVI | PIND-ORF | Kombination AVI/ORF | BCG | Levamisol | C.parvum |
| 1:4 | 77 | 93 | 90 | 21 | toxisch | |
| 1:8 | 47 | 78,5 | 96 | 0 | 0 | toxisch |
| 1:16 | 40.5 | 76.5 | 86 | 0 | 0 | 22 |
| 1:32 | 8.5 | 63 | 70 | - | 0 | 21 |
| 1:64 | 0 | 27 | 56 | - | 0 | 0 |
| 1:128 | 0 | 1.5 | 27.5 | - | 0 | 0 |
| 1:256 | 0 | 0 | 4 | - | - | 0 |

EP 0 669 133 B1

Tabelle 2:

| Virusstamm | WE/ml im VSV-Test | | |
|---|---|---|---|
| | Wildtyp | niedrige Attenu-ierungspassage | hohe Attenu-ierungspassage |
| Vaccinevirus MVA | < 20 | $\leqq 40$ | 160 - 320 |
| Hühnerpocken-virus HP 1 | < 20 | $\leqq 40$ | 80 - 160 |
| Kanarienp.-virus KP 1 | < 20 | $\leqq 40$ | 160 - 320 |
| Parapocken-virus ORF D 1701 | < 20 | $\leqq 40$ | 320 - 640 |

Tabelle 3

| Virusspezies | Stamm | Inf.titer -log10 (MOI = 1) | antivirale Aktivität in E.U./ml mononukleärer Leukozyten von | | |
| --- | --- | --- | --- | --- | --- |
| | | | Mensch | Schaf | Schwein |
| Vacciniavirus Wildtyp | Kopen- hagen | 8,75 | < 40 | < 40 | < 40 |
| niedr.Pass. | Elstree | 7.4 | < 40 | < 40 | n.u. |
| | Bern | 7.05 | < 40 | < 40 | < 40 |
| attenuiert | MVA | 8.05 | 160-320 | 80 | 320 |
| Hühner- niedrige pocken- Passage virus | HP1 | 7.0 | < 40 | < 40 | < 40 |
| attenuiert | | 6.3 | 1280 | 320 | 80 |
| Para- Wild- pocken- typ virus attenuiert | D 1701 | 6.5 | < 40 | < 40 | < 40 |
| | | 7.3 | 320-640 | 160 | 160 |

Tabelle 4

| Inducertyp | WE/ml<br>(Vorverdünnung 1:8) |
|---|---|
| PIND-AVI | 160 - 320 |
| PIND-ORF | 320-640 |
| ORF-Protein<br>4D9 | 40 -80 |
| rekombinierter Para-<br>munitätsinducer<br>PIND-AVI<br>+ ORF 4D9 | 1280 - 2560 |

## Abbildung 1:

## Abbildung 2:

% spez.Lysis
(Effektor/
Zielzellen
YAC-1
= 100:1)

Bar chart with y-axis labeled from 2 to 36. Bars: PIND-AVI (~15), ORF-Protein 4d9 (~14), Kombination PIND-AVI/ORF-Protein 4D9 (~33).